# EUROPEAN PATENT SPECIFICATION

(11) **EP 0 808 314 B1**
(45) Date of publication and mention of the grant of the patent: **12.06.2002**
(21) Application number: 96903970.0
(22) Date of filing: 02.02.1996
(51) Int. Cl.: C07D 405/12

(54) **FORMS OF PAROXETIN HYDROCHLORIDE**
FORMEN VON PAROXETIN HYDROCHLORID
FORMES DE L'HYDROCHLORURE DE PAROXETINE

(30) Priority: 06.02.1995 GB 9502297; 17.02.1995 GB 9503112; 15.05.1995 GB 9509807
(43) Date of publication of application: 26.11.1997
(60) Divisional application: 01115079.4
(73) Proprietor: SMITHKLINE BEECHAM PLC, Brentford, Middlesex TW8 9EP (GB)
(72) Inventor: JACEWICZ, Victor Witold, SmithKline Beecham, Tonbridge Kent TN11 9AN (GB); WARD, Neal SmithKline Beecham Pharmaceuticals, Kent TN11 9AN (GB)
(74) Representative: West, Vivien
(86) International application number: EP9600447
(87) International publication number: WO9624595

(56) References cited:
- EP-A- 0 223 403
- INTERNATIONAL JOURNAL OF PHARMACEUTICS, vol. 42, 1988, AMSTERDAM NL, pages 135-143, XP000572028 P. CHRISTOPHER BUXTON ET AL. : "Solid-State Forms of Paroxetine Hydrochloride" cited in the application
- Priority of the British application 85/26407
- : "", ANALYTICAL PROCEEDINGS, , 1988, vol. 25, no. , pages 305 to 306

## Description

The present invention relates to novel compounds, to processes for preparing them and to their use in treating medical disorders.

EP-B-223403 (Beecham Group plc) and UK patent Application No. 8 526 407 from which it claims priority describe paroxetine hydrochloride hemihydrate and its use in treating certain medical disorders. Example 8 in EP-B-223 403 describes the preparation of paroxetine hydrochloride anhydrate as platelets melting at 118°C and with IR bands at 890, 1200, 1490, 3400 and 3640cm⁻¹, by crystallisation from a water-containing solvent. Subsequent repetition of the preparation described in these two documents has failed to yield any type of paroxetine hydrochloride anhydrate.

Paroxetine hydrochloride anhydrate is also purported to be disclosed in the International Journal of Pharmaceutics 42, (1988) 135 to 143, published by Elsevier and in Analytical Proceedings Sept. 1988 25 305-306. The anhydrate is said to be produced by crystallising paroxetine hydrochloride from anhydrous propan-2-ol. Subsequent repetition of this process has resulted in a propan-2-ol solvate of paroxetine hydrochloride. That is to say that there is bound propan-2-ol in the product. This bound propan-2-ol cannot be removed by conventional drying techniques such as vacuum oven drying.

A new form of paroxetine hydrochloride anhydrate has now been found as have processes for its preparation.
This form is hereinafter referred to as Form C. The characterising data for the Form C do not correspond to the characterising data provided in Example 8 of EP-A-223403.

Accordingly, the present invention provides paroxetine hydrochloride anhydrate in Form C as claimed in claim 1.

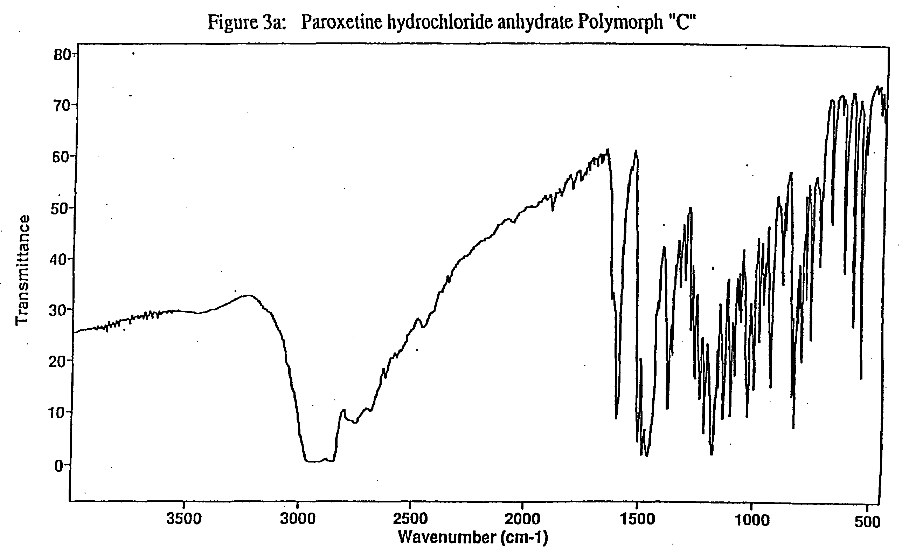

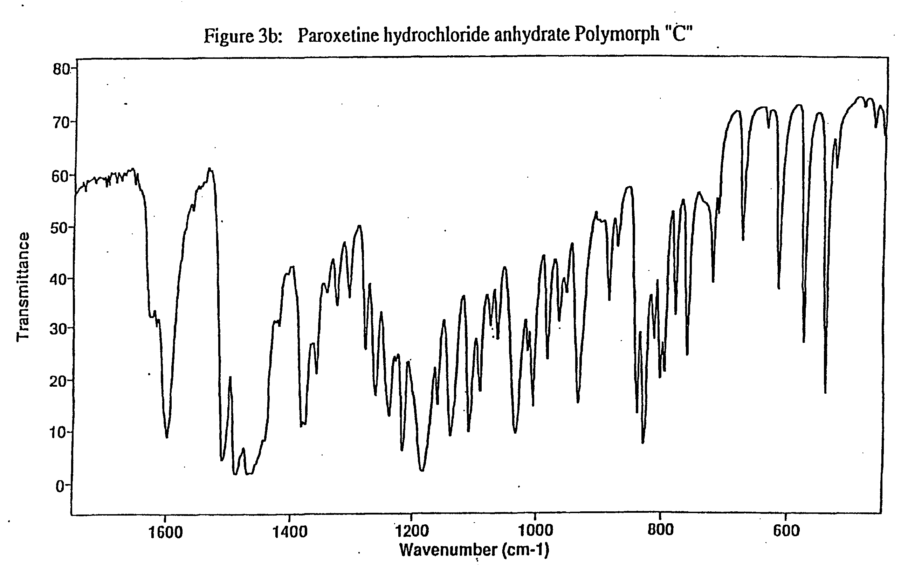

Form C may be distinguished from other forms by the following analytical data, i.e. it has a melting point of about 164°C when obtain in similar purity to the material described in Example 1 which may be determined by conventional methods such as HPLC and has significant IR bands (Figure 3) at about 540, 574, 615, 674, 720, 760, 779, 802, 829, 840, 886, 935, 965, 984, 1007, 1034, 1092, 1109, 1139, 1183, 1218, 1240, 1263, 1280, 1507, 1540, 1558, 1598, 1652 cm⁻¹.

The DSC exotherm, measured at 10°C per minute, shows a maximum of about 161°C in both open and closed pans.

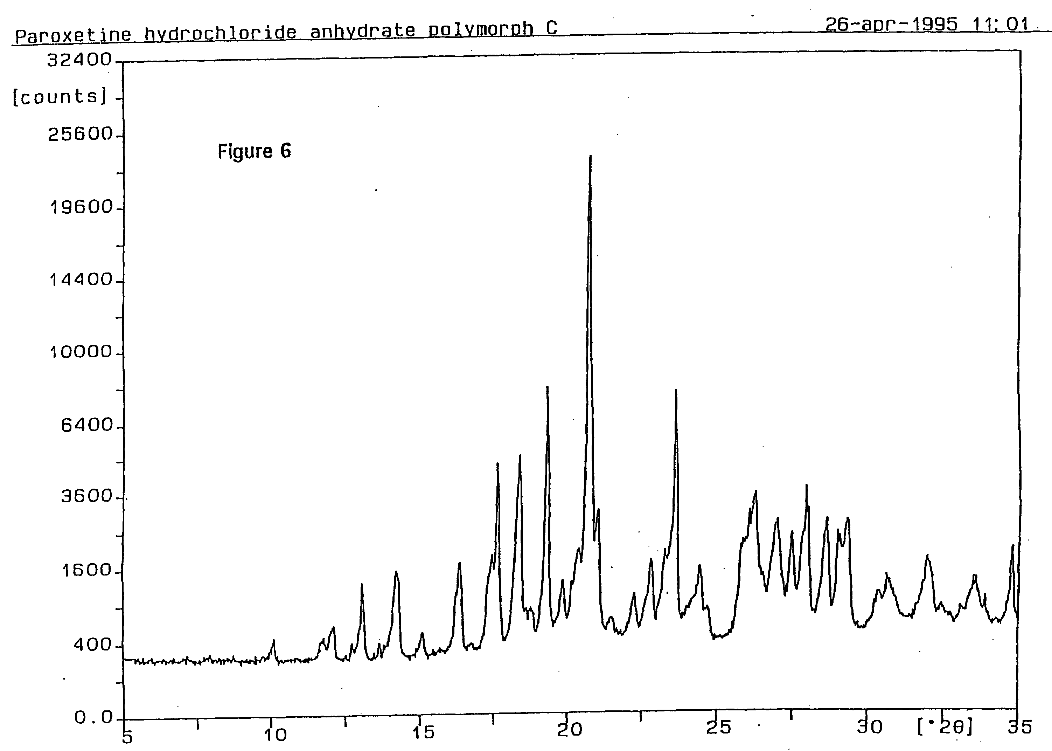

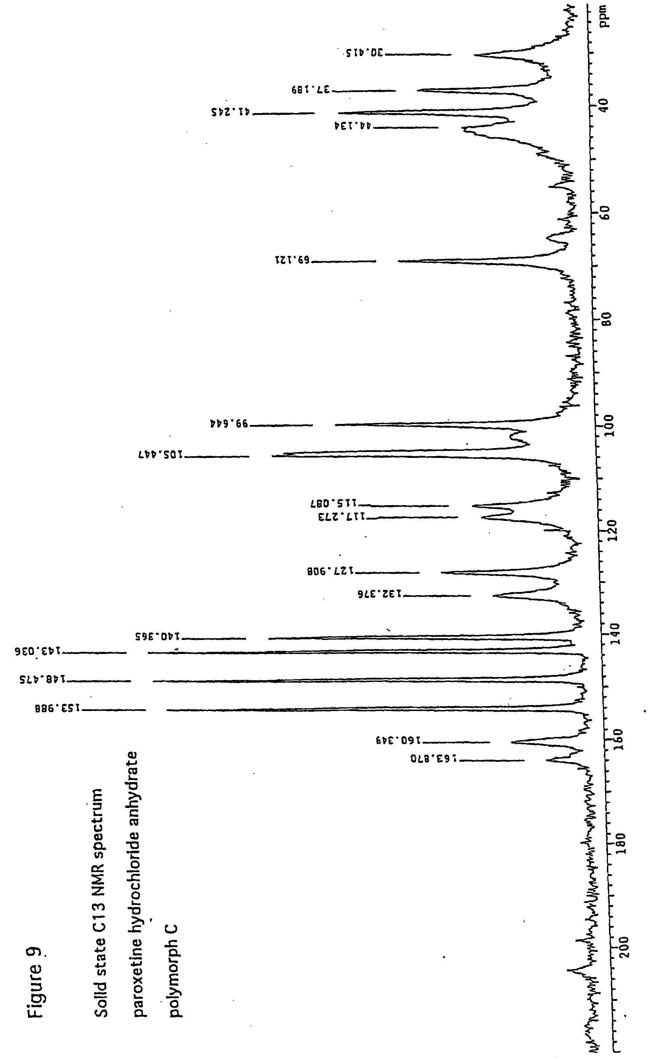

Form C also has a substantially similar X-ray diffractogram to that shown in Figure 6, for example there are characteristic peaks at 10.1, 12.1, 13.1, 14.3 degrees 2 theta and a substantially similar solid state NMR spectrum to that in Figure 9, for example with characteristic peaks at 154.0, 148.5, 143.4, 140.4 ppm.

Preferably form C exists as needles or prisms.

The present invention also provides a process for the preparation of paroxetine hydrochloride anhydrate Form C which comprises crystallising paroxetine hydrochloride in an organic solvent or mixture of organic solvents selected from the group consisting of butan-1-ol, ethyl acetate, toluene and 2-butanone.

In one preferred aspect of the invention crystallisation of paroxetine hydrochloride anhydrate is achieved by contacting a solution of paroxetine free base in the organic solvent or solvents with dry hydrogen chloride gas.

Paroxetine hydrochloride free base may be prepared according to the procedures generally outlined in EP-B-0 223403.

The organic solvents should be substantially free of water to the extent that there is insufficient water present at the time of crystallisation to effect conversion to the hydrochloride hemi-hydrate. Organic solvents which are substantially free of water may be obtained in conventional manner. For example they can be dried using conventional techniques such as drying over molecular sieves or they can be purchased.

It should also be appreciated that the use of seeds of Form C may be used to facilitate the crystallisation of Form C.

Seed crystals of Form C may be prepared according to the procedures described herein or are freely available upon request to Corporate Intellectual Property, SmithKline Beecham plc at New Frontiers Science Park, Third Avenue, Harlow, Essex, CM19 5AW, United Kingdom. Form C is BRL.

Paroxetine hydrochloride anhydrate in Form C (which is hereinafter referred to as the "product of the invention") can be used to treat and prevent the following disorders:
Alcoholism
Anxiety
Depression
Obsessive Compulsive Disorder
Panic Disorder
Chronic Pain
Obesity
Senile Dementia
Migraine
Bulimia
Anorexia
Social Phobia
Pre-Menstrual Syndrome (PMS)
Adolescent Depression
Trichotillomania
Dysthymia
Substance Abuse

These disorders are herein after referred to as "the Disorders".

The present invention further provides a pharmaceutical composition for use in the treatment and/or prevention of the Disorders which comprises the product of the invention with a pharmaceutically acceptable carrier.

The present invention also provides the use of the product of the invention in the manufacture of a medicament for treating and/or preventing the Disorders.

Preferred disorders include depression, OCD and panic.

The composition of this invention is usually adapted for oral administration, but formulations for dissolution for parental administration are also within the scope of this invention.

The composition is usually presented as a unit dose composition containing from 1 to 200mg of active ingredient calculated on a free base basis, more usually from 5 to 100mg, for example 10 to 50mg such as 10, 12.5, 15, 20, 25, 30 or 40mg by a human patient. Most preferably unit doses contain 20mg of active ingredient calculated on a free base basis. Such a composition is normally taken from 1 to 6 times daily, for example 2, 3 or 4 times daily so that the total amount of active agent administered is within the range 5 to 400mg of active ingredient calculated on a free base basis. Most preferably the unit dose is taken once a day.

Preferred unit dosage forms include tablets or capsules.

The compositions of this invention may be formulated by-conventional methods of admixture such as blending, filling'and compressing.

Suitable carriers for use in this invention include a diluent, a binder, a disintegrant, a colouring agent, a flavouring agent and/or preservative. These agents may be utilised in conventional manner, for example in a manner similar to that already used for marketed antidepressant agents.

Specific examples of pharmaceutical compositions include those described EP-B-0-223403, and US 4,007,196 in which the products of the present invention are used as the active ingredients.

The following examples illustrate the present invention:

### DESCRIPTION 1

### Use in the preparation of form C

### Crystalline Paroxetine Hydrochloride Anhydrate Substantially Free of Bound Propan-2-ol (Form A)

### i) Paroxetine hydrochloride propan-2-ol solvate

Paroxetine hydrochloride hemihydrate [150 g] was stirred with propan-2-ol [1000 ml] and toluene [300 ml] in a round bottom flask and heated to boiling. Solvent was removed by distillation, the total volume being maintained by adding fresh propan-2-ol, until the boiling point had reached approximately 82°C, indicating that all the water had been removed.

The mixture was allowed to cool to approximately 50°C, when it spontaneously crystallised. The contents of the flask rapidly set to a thick paste which was diluted with propan-2-ol [approx. 500 ml] and stirred vigorously. The resulting suspension was allowed to cool-to approximately 30°C and filtered under vacuum, taking care to avoid the absorption of atmospheric moisture. The solvent-wet cake was dried in high vacuum over phosphorus pentoxide.

Yield of solvated paroxetine hydrochloride 151 g, propan-2-ol content 13.0% (estimated by NMR).

The infra-red spectrum (Nujol mull) showed *inter alia* a characteristic band at 667 cm⁻¹.

### ii) Paroxetine hydrochloride anhydrate (Form A)

Paroxetine hydrochloride propan-2-ol solvate [110 g, propan-2-ol content 13.0%] was stirred in a beaker with water [275 ml] for 20 minutes. The mixture was filtered under vacuum and the damp solid dried in vacuum over phosphorus pentoxide to constant weight.

Yield of paroxetine hydrochloride anhydrate Form A 91.0 g.

Water content 0.13% (KF), propan-2-ol content 0.05% (estimated by NMR).

Melting point: 123-125°C.

The DSC exotherm, measured at 10°C per minute, showed a maximum at about 126°C using an open pan and a maximum at about 121°C using a closed pan.

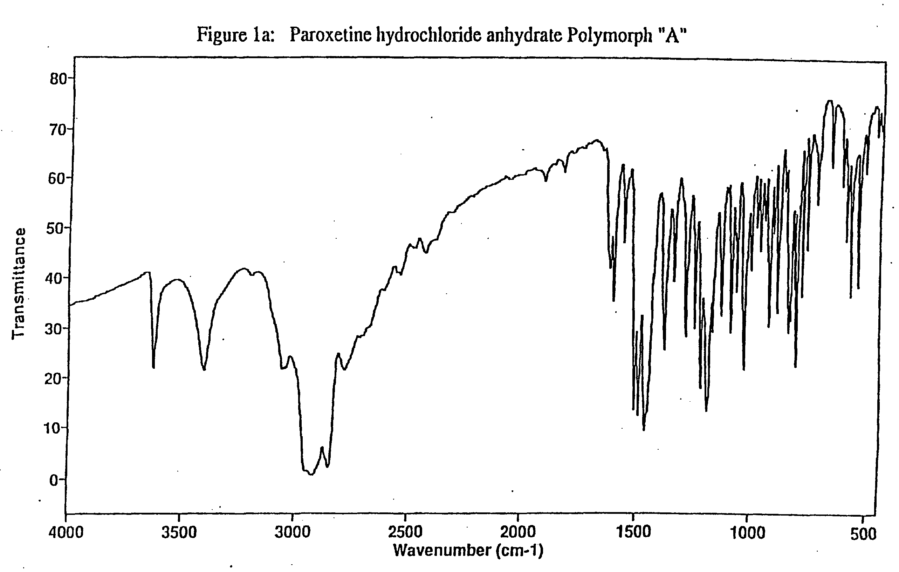

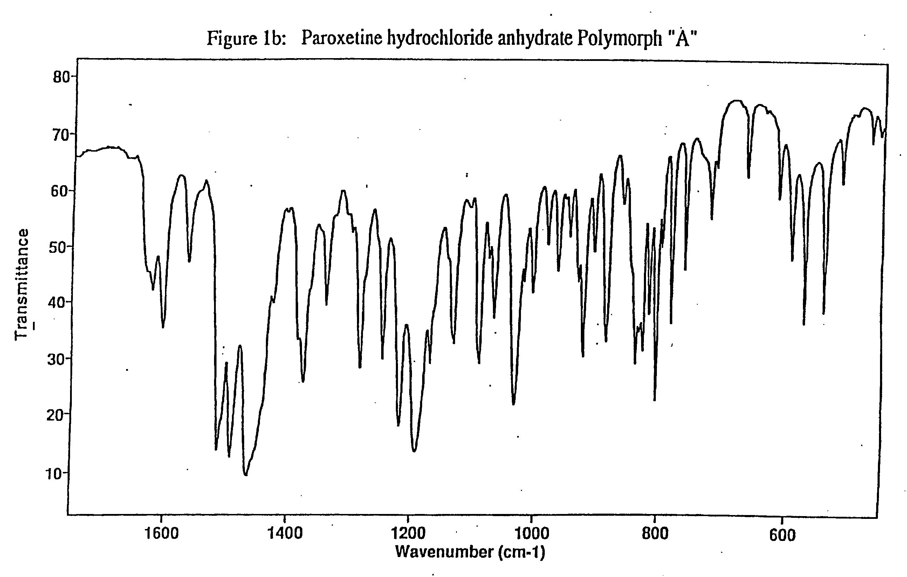

The infra-red spectrum [Nujol mull] showed *inter alia* characteristic bands at 665, 3631 and 3402 cm⁻¹ (see Figure 1).

| Elemental analysis: | | | |
|---|---|---|---|
| Requires for paroxetine hydrochloride anhydrate | C 62.38 | H 5.79 | N 3.83% |
| Found | C 62.10 | H 5.89 | N 3.67% |

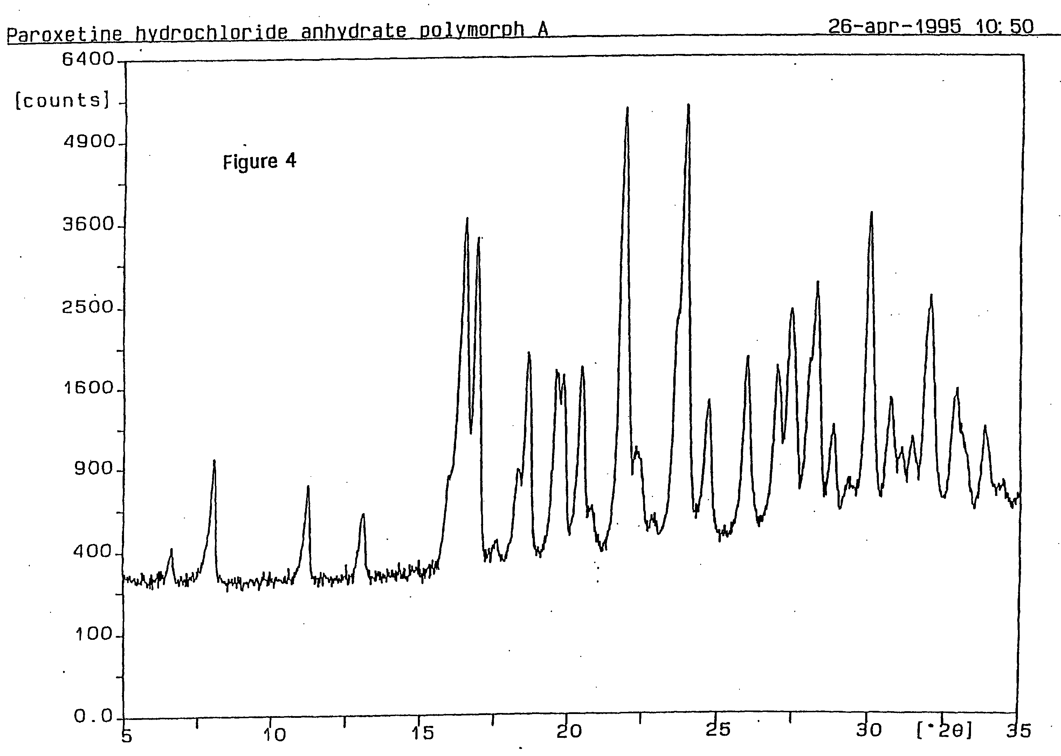

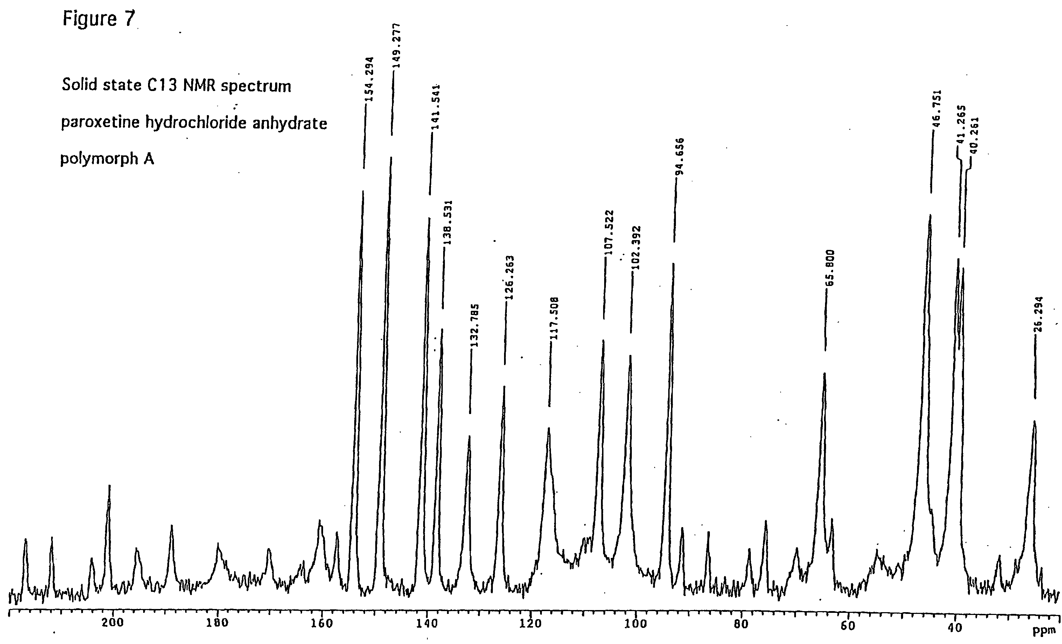

The sample was also examined by X-ray powder diffraction (see Figure 4) and solid state C13 NMR (see Figure 7).

### DESCRIPTION 2

### Paroxetine Hydrochloride Anhydrate (Form B)

Paroxetine free base [10.0 g] was dissolved in butan-1-ol [25 ml] at room temperature and a solution of hydrogen chloride gas [1.25 g] in butan-1-ol [15 ml] was added. The clear pale red/brown solution was sealed and stored in the refrigerator overnight. A small amount of crystalline material formed on the base of the flask and ultrasonication was used to bring about crystallisation of the bulk. The mixture was again stored in the refrigerator overnight, then allowed to warm to room temperature and filtered. The product was dried under high vacuum in a desiccator containing phosphorus pentoxide.

Microscopic exarnination with a polarising microscope showed the sample to be in the form of feather shaped crystals.

Melting point: 137-138°C.

The NMR (CDCl₃) spectrum conformed to that of a standard sample of paroxetine hydrochloride.

The elemental analysis was consistent with anhydrous paroxetine hydrochloride:

| | | | | |
|---|---|---|---|---|
| Required for C₁₉H₂₁NClFO₃ | C 62.38 | H 5.79 | N 3.83 | Cl 9.69% |
| Found | C 62.08 | H 5.75 | N 3.81 | Cl 9.62% |

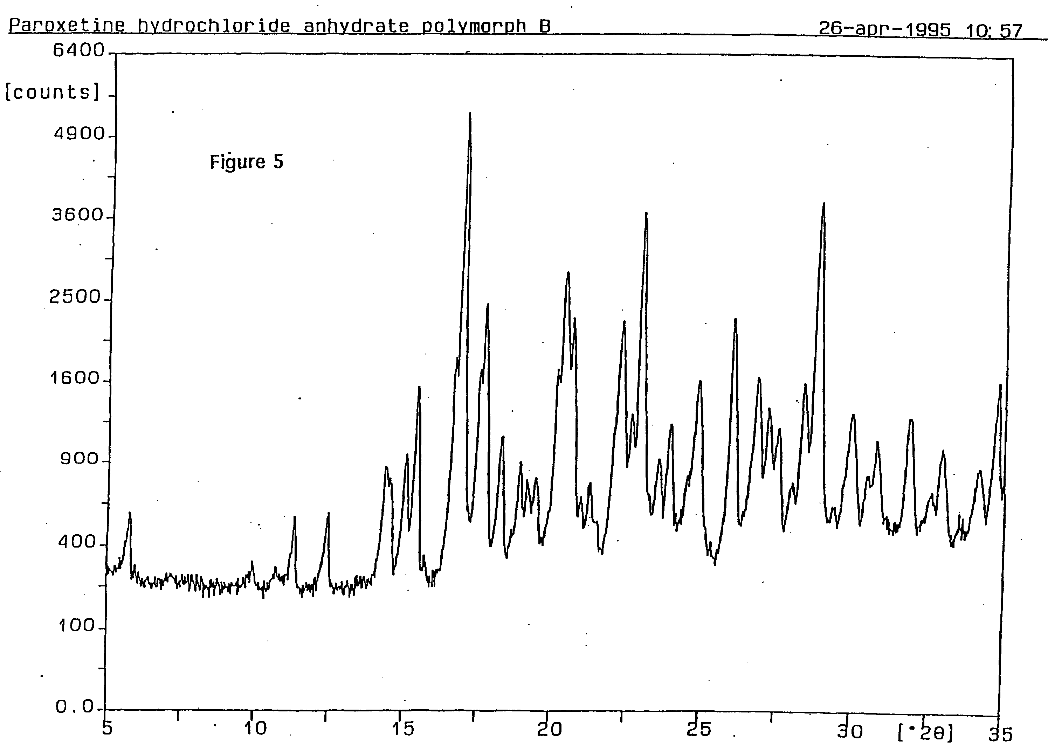

The X-ray powder diffractogram confirmed that the sample was crystalline (see Figure 5). The diffractogram differed from both that of hemihydrate and anhydrate Form A.

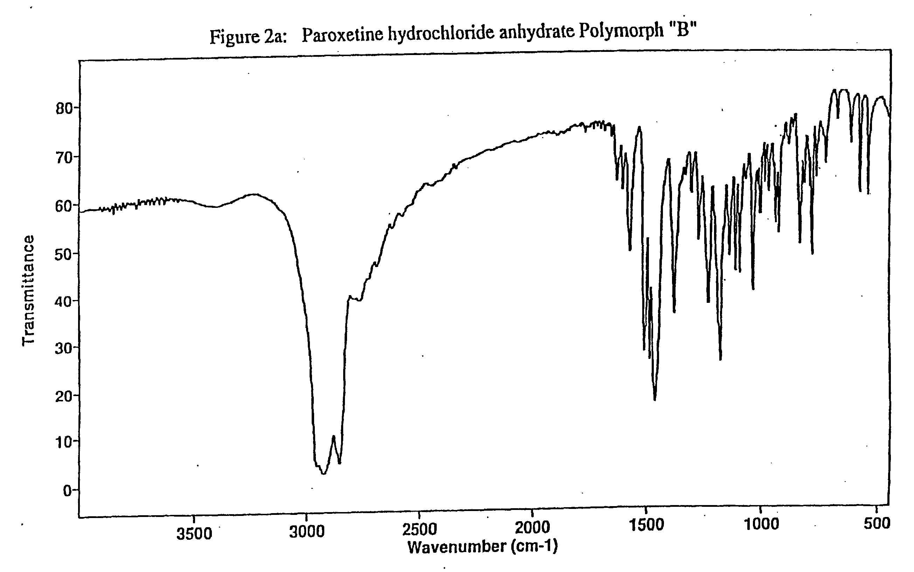

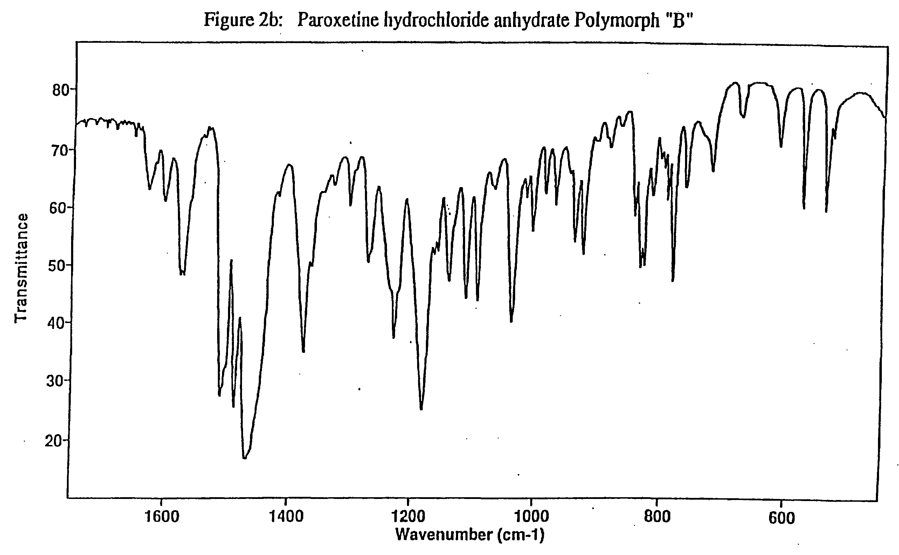

The IR spectrum (Nujol mull) also differed from both that of hemihydrate and anhydrate Form A (see Figure 2).

The DSC exotherm, measured at 10°C per minute, showed a maximum at about 137°C in both open and closed pans.

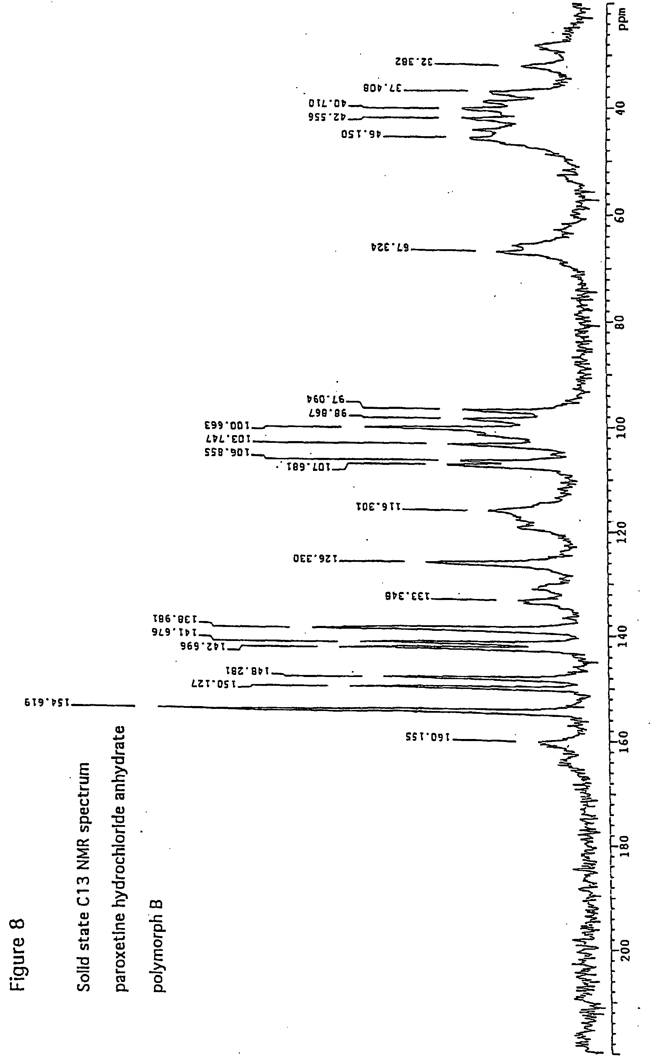

The sample was also examined by solid state C13 NMR (see Figure 8).

### EXAMPLE 1

### Paroxetine Hydrochloride Anhydrate (Form C)

Paroxetine hydrochloride hemihydrate [300 g] and toluene [1200 ml] were heated under reflux and the water removed using a Dean and Stark apparatus. When no further water could be collected, the bulk of the toluene was removed by distillation and replaced with anhydrous butan-1-ol. Distillation was continued until the still temperature reached about 117°C, indicating that all the toluene had been removed. The mixture was diluted to about 1200 ml with butan-1-ol and allowed to cool. At about 42°C, seeds of paroxetine hydrochloride anhydrate Form B (needles) were added. Although crystallisation then began, it was observed that the product was in the form of well formed prisms, indicating that the product was crystallising in a different form to the seeds added.

The mixture was allowed to stand overnight, then filtered. The crystals were washed on the filter with butan-1-ol, then dried in vacuum at 50°C over phosphorus pentoxide.
Yield 250 g,
Melting point: 162-164ºC
Analysis by NMR (CDCl₃) confirmed that the product was paroxetine hydrochloride and showed the presence of a trace of butan-1-ol (ca 0.1% by weight). The infra red spectrum (Nujol mull) differed from either Form A or B, (see Figure 3),
Water content 0.06% (KF)

The elemental analysis was consistent with anhydrous paroxetine hydrochloride:

| | | | | |
|---|---|---|---|---|
| Required for C₁₉H₂₁NClFO | C 62.38 | H 5.79 | N 3.83 | Cl 9.69% |
| Found | C 62.23 | H 5.67 | N 3.83 | Cl 9.74% |

The DSC exotherm, measured at 10°C per minute, showed a maximum at about 161°C in both open and closed pans.

The X-ray powder diffractogram confirmed that the sample was crystalline (see Figure 6). The diffractogram differed from both that of anhydrate Form A and anhydrate Form B.

The sample was also examined by solid state C13 NMR (see Figure 9).

### EXAMPLE 2

### Paroxetine Hydrochloride Anhydrate (Form C)

Paroxetine free base (8.5 g) was dissolved in ethyl acetate (40 ml) and hydrogen chloride gas was bubbled in until the weight of the flask and contents had increased by 1.1 g. There was no sign of crystallisation after 15 minutes, so the clear solution was seeded with paroxetine hydrochloride anhydrate Form A. After stirring for a further one hour, signs of very slow crystallisation could be seen. The mixture was left stirring overnight to crystallise in a stoppered flask, then filtered and dried at ambient temperature in a vacuum desiccator containing phosphorus peptoxide.

Weight of product: 7.56 g. Ethyl acetate content (estimated by NMR) 0.4% wt/wt. The infra-red spectrum was different from both paroxetine hydrochloride hemihydrate and anhydrate Form A and consistent with the infra-red spectrum obtained in Example 8.

### EXAMPLE 3

### Crystallisation of paroxetine hydrochloride anhydrate Form C from 2-butanone by seeding

Paroxetine hydrochloride anhydrate Form C [7.0 g] was heated to boiling in anhydrous 2-butanone [40 ml] and the solution allowed to cool to ca 40°C. Seeds of Form C were added and the stirred mixture allowed to cool to room temperature. The product was collected by filtration, washed with anhydrous 2-butanone [20 ml] and dried in an oven at 100°C.

| | |
|---|---|
| Weight of dried product | 5.95 g |
| Melting point | 162-163°C |

The infra-red spectrum (Nujol mull) was consistent with paroxetine hydrochloride anhydrate Form C.

### EXAMPLE 4

### Crystallisation of paroxetine hydrochloride from toluene by seeding

Paroxetine hydrochloride anhydrate Form C [20.0 g] was dissolved in boiling toluene [200 ml] and approximately 50 ml of the solution added to each of 2 conical flasks. Each flask was heated again to boiling, allowing some toluene vapour to reflux out, in order to remove seeds. Flask 1 was immediately sealed with a ground glass stopper and set aside to cool. The remaining fiask was sealed with foil, and allowed to cool somewhat, before adding seed crystals of paroxetine hydrochloride anhydrate Form C.

The added seeds remained undissolved. The flasks were sealed with ground glass stoppers, stirred gently for a few seconds then set aside to cool. At this point Flask 1 remained completely clear, and the flasks were left at room temperature overnight. The following morning Flask 1 contained only a few crystals, while Flask 2 had extensively crystallised.

Flask 1 was stirred gently for several hours, during which time the bulk of the paroxetine hydrochloride crystallised.

The product from each flask was collected by filtration and dried at 50°C under vacuum.

| Flask 1 (not seeded) | |
|---|---|
| Weight of product | 4.25 g |
| Appearance | short needles/rods |
| Infra red spectrum | consistent with paroxetine hydrochloride anhydrate Form C |
| Melting point | 161 - 162°C |

| Flask 2 (seeded with anhydrate Form C) | |
|---|---|
| Weight of product | 4.93 g |
| Appearance | needles |
| Infra red spectrum | consistent with paroxetine hydrochloride anhydrate Form C |
| Solvent content | 0.8% wt/wt toluene by NMR |
| Melting point | 161 - 162°C |

## Claims

1. Paroxetine hydrochloride anhydrate in Form C; which is **characterised in that** it has a melting point of about 164°C and it has significant IR bands (Nujol Mull) at about 540, 574, 615, 674, 720, 760, 779, 802, 829, 840, 886, 935, 965, 984, 1007, 1034, 1092, 1109, 1139, 1183, 1218, 1240, 1263, 1280, 1507, 1540, 1558, 1598, 1652 cm⁻¹; the DSC exotherm, measured at 10°C per minute, shows a maximum of about 161°C in both open and closed pans; it also has a X-ray diffractogram including characteristic peaks at 10.1, 12.1, 13.1, 14.3 degrees 2 theta and a solid state NMR spectrum including characteristic peaks at 154.0, 148.5, 143.4, 140.4 ppm.

2. Paroxetine hydrochloride anhydrate as defined in claim 1, which is in the form of needles or prisms.

3. A process for the preparation of paroxetine hydrochloride anhydrate Form C, which comprises crystallising paroxetine hydrochloride in an organic solvent or mixture of organic solvents selected from the group consisting of butan-1-ol, ethyl acetate, 2-butanone and toluene.

4. A pharmaceutical composition comprising a compound as claimed in claim 1or 2 and a pharmaceutically acceptable carrier.

5. The use of a compound as claimed in claim 1 or 2 in the manufacture of a medicament for the treatment and/or prevention of the Disorders as defined herein.

## Patentansprüche

1. Paroxetinhydrochloridanhydrat in Form C, **dadurch gekennzeichnet, dass** es einen Schmelzpunkt von etwa 164°C und signifikante IR-Banden (Nujolfilm) bei etwa 540, 574, 615, 674, 720, 760, 779, 802, 829, 840, 886, 935, 965, 984, 1007, 1034, 1092, 1109, 1139, 1183, 1218, 1240, 1263, 1280, 1507, 1540, 1558, 1598, 1652 cm⁻¹ aufweist, die bei 10°C pro Minute gemessene DSC-Exotherme sowohl in offenen als auch geschlossenen Schalen ein Maximum von etwa 161°C zeigt, es auch ein Röntgenbeugungsdiagramm, umfassend charakteristische Signale bei 10,1, 12,1, 13,1, 14,3° 2θ, und ein Festphasen-NMR-Spektrum, umfassend charakteristische Signale bei 154,0, 148,5, 143,4, 140,4 ppm, zeigt.

2. Paroxetinhydrochloridanhydrat nach Anspruch 1, das in Form von Nadeln oder Prismen vorliegt.

3. Verfahren zur Herstellung von Paroxetinhydrochloridanhydrat Form C, das das Kristallisieren von Paroxetinhydrochlorid in einem organischen Lösungsmittel oder einem Gemisch aus organischen Lösungsmitteln, ausgewählt aus Butan-1-ol, Essigsäureethylester, 2-Butanon und Toluol, umfasst.

4. Arzneimittel, umfassend eine Verbindung nach Anspruch 1 oder 2 und einen pharmazeutisch verträglichen Träger.

5. Verwendung einer Verbindung nach Anspruch 1 oder 2 bei der Herstellung eines Medikaments zur Behandlung und/oder Vorbeugung der hier definierten Erkrankungen.

## Revendications

1. Anhydrate de chlorahydrate de paroxétine sous la forme C, qui es **caractérisé en ce qu'**il a un point de fusion d'environ 164°C et il présente des bandes de IR significatives (pâte de Nujol) à environ 540, 574, 615, 674, 720, 760, 779, 802, 829, 840, 886, 935, 965, 984, 1007, 1034, 1092, 1109, 1139, 1183, 1218, 1240, 1263, 1280, 1507, 1540, 1558, 1598, 1652 cm⁻¹ ; le dégagement de chaleur par DSC, mesuré à 10°C par minute, présente un maximum d'environ 161°C à la fois dans des récipients ouverts et des récipients clos ; il présente également un diffractogramme des rayons X comprenant des pics caractéristiques à 10,1, 12,1, 13,1, 14,3 degrés 2 thêta et un spectre de RMN à l'état solide comprenant des pics caractéristiques à 154,0, 148,5, 143,4 et 140,4 ppm.

2. Anhydrate de chlorhydrate de paroxétine répondant à la définition suivant la revendication 1, qui est sous forme d'aiguilles ou de prismes.

3. Procédé pour la préparation de l'anhydrate de chlorhydrate de paroxétine sous la forme C, qui comprend la cristallisation de chlorhydrate de paroxétine dans un solvant organique ou mélange de solvants organiques choisis dans le groupe consistant en l'acétate d'éthyle, la 2-butanone et le toluène.

4. Composition pharmaceutique comprenant un composé suivant la revendication 1 ou 2, et un support pharmaceutiquement acceptable.

5. Utilisation d'un composé suivant la revendication 1 ou 2, dans la production d'un médicament destiné au traitement et/ou à la prévention des Affections définies dans le présent mémoire.
